# EUROPEAN PATENT APPLICATION

(11) **EP 4 015 041 A1**
(43) Date of publication of application: **22.06.2022**
(21) Application number: 20383097.1
(22) Date of filing: 15.12.2020
(51) Int. Cl.: A61P 31/10, C07K 16/14

(54) **TREATMENT OF FUNGAL INFECTIONS OR DISEASES**

(71) Applicant: Universidad De Zaragoza, 50009 Zaragoza (ES); Fundación Agencia Aragonesa Para La Investigación Y El Desarrollo (ARAID), 50018 Zaragoza (ES); Universidad Complutense De Madrid, 28040 Madrid (ES); Vrije Universiteit Brussel, 1050 Brussel (BE); Consejo Superior De Investigaciones Científicas, 28006 Madrid (ES)
(72) Inventor: HURTADO GUERRERO, Ramón, 50009 Zaragoza (ES); PARDO JIMENO, Julián, 50009 Zaragoza (ES); GALVEZ BUERBA, Eva María, 50018 Zaragoza (ES); ARROYO NOMBELA, Francisco Javier, 28040 Madrid (ES); MUYLDERMANS, Serge, 1050 Brussels (BE)
(74) Representative: Hoffmann Eitle

(57) **Abstract**

The present invention refers to the treatment of fungal infections or diseases by using specific affinity reagents, such as nanobodies, which specifically bind the fungal β-1,3-glucanosyltransferase gel4.

SEQUENCE LISTING

<110>  Universidad de Zaragoza
       Fundación Agencia Aragonesa para la Investigación y el Desarrollo
(ARAID)
       Universidad Complutense de Madrid
       Vrije Universiteit Brussel
       Consejo Superior de Investigaciones Cientificas

<120>  TREATMENT OF FUNGAL INFECTIONS OR DISEASES

<130>  905734

<160>  15

<170>  PatentIn version 3.5

<210>  1
<211>  548
<212>  PRT
<213>  Aspergillus fumigatus Gel4

<400>  1

Met Lys Phe Val Tyr Ala Ala Ala Gly Ala Ser Leu Val Gly Ser Ala
1               5                   10                  15

Leu Ala Thr Leu Pro Val Ile Glu Ala Lys Gly Asn Lys Phe Phe Tyr
            20                  25                  30

Ser Asn Asn Gly Thr Glu Phe Phe Ile Arg Gly Val Ala Tyr Gln Gln
        35                  40                  45

Glu Tyr Gln Ala Asn Gly Thr Ser Thr Glu Asn Ser Asp Tyr Thr Asp
    50                  55                  60

Pro Leu Ala Asn Val Asp Asn Cys Lys Arg Asp Ile Pro Tyr Leu Lys
65                  70                  75                  80

Gln Leu Arg Thr Asn Val Ile Arg Thr Tyr Ala Val Asp Pro Thr Lys
                85                  90                  95

Asp His Asp Glu Cys Met Lys Leu Leu Asp Asp Ala Gly Ile Tyr Leu
            100                 105                 110

Ile Thr Asp Leu Ser Ala Pro Ser Glu Ser Ile Asn Arg Ala Asp Pro
        115                 120                 125

Ala Trp Asn Thr Asp Leu Tyr Lys Arg Tyr Thr Ser Val Ile Asp Ala
    130                 135                 140

Phe Ala Lys Tyr Ser Asn Val Ile Gly Phe Phe Ala Gly Asn Glu Val
145                 150                 155                 160

Ala Asn Asp Asn Asn Asn Thr Asn Ser Ile Ala Tyr Val Lys Ala Ala
                165                 170                 175

Val Arg Asp Met Lys Ser Tyr Ile Lys Ser Lys Asp Tyr Arg Ser Ser
            180                 185                 190

Leu Leu Val Gly Tyr Ala Thr Asp Asp Asp Ala His Ile Arg Ala Asp
        195                 200                 205

Leu Ala Asp Tyr Leu Val Cys Gly Asp Lys Glu Ser Ser Ile Asp Met
    210                 215                 220

Phe Gly Tyr Asn Ile Tyr Glu Trp Cys Gly Asp Ser Ser Phe Glu Lys
225                 230                 235                 240

Ser Gly Tyr Lys Asp Arg Thr Glu Glu Phe Ser Lys Tyr Pro Val Pro
                245                 250                 255

Ala Phe Phe Ser Glu Tyr Gly Cys Ile Asp Pro Lys Pro Arg Lys Phe
            260                 265                 270

Thr Asp Val Ala Ala Leu Tyr Gly Pro Gln Met Asn Asp Val Trp Ser
        275                 280                 285

Gly Gly Ile Val Tyr Met Tyr Phe Gln Glu Ala Asn Asp Tyr Gly Leu
    290                 295                 300

Val Ser Val Ser Gly Asp Asn Val Lys Thr Lys Glu Asp Phe Ser Tyr
305                 310                 315                 320

Leu Ser Val Gln Met Gln Lys Val Thr Ala Thr Gly Val Asn Ser Ala
                325                 330                 335

Ser Tyr Thr Ala Ser Asn Thr Ala Val Pro Thr Cys Pro Ser Val Gly
            340                 345                 350

Ala Lys Trp Glu Ala Ser Asn Lys Leu Pro Pro Ser Pro Asn Ser Glu
        355                 360                 365

Leu Cys Asp Cys Met Val Glu Thr Leu Ser Cys Thr Val Lys Asp Ser
    370                 375                 380

Val Asp Glu Lys Glu Tyr Gly Asp Leu Phe Asp Tyr Leu Cys Ala Ala
385                 390                 395                 400

Gly Val Cys Gly Gly Ile Asn Ser Asn Ser Thr Ser Gly Asp Tyr Gly
                405                 410                 415

Ala Tyr Ser Val Cys Ser Ala Lys Gln Lys Leu Ser Phe Val Met Asn
                420                 425                 430

Gln Tyr Tyr Lys Lys Asn Asn Lys Ala Ala Thr Ala Cys Asp Phe Asp
            435                 440                 445

Gly Lys Ala Gln Thr Lys Lys Gly Ala Asp Ala Ser Gly Ser Cys Ala
    450                 455                 460

Ser Leu Ile Ser Gln Ala Gly Thr Ala Gly Thr Gly Ser Val Thr Ala
465                 470                 475                 480

Gly Ala Thr Gly Ser Ser Gly Ser Gly Ser Ala Ser Glu Thr Ser Lys
                485                 490                 495

Gly Ala Ala Gly Val Ala Ala Ser Pro Met Ala Val Lys Val Gly Asn
            500                 505                 510

Trp Gln Phe Gly Ala Tyr Ile Ala Thr Ala Leu Phe Ala Gly Val Gly
        515                 520                 525

Met Leu Val Leu
    530

<210>  2
<211>  8
<212>  PRT
<213>  Artificial sequence

<220>
<223>  Artificial sequence

<400>  2

Gly Asn Ile Tyr Ser Thr Asn Ala
1               5

<210>  3
<211>  8
<212>  PRT
<213>  Artificial sequence

<220>
<223>  Artificial sequence

<400>  3

Ile Thr Arg Gly Gly Arg Thr Asn
1               5

<210>  4

<211>  15
<212>  PRT
<213>  Artificial sequence

<220>
<223>  Artificial sequence

<400>  4

Asn Val Glu Val Arg Thr Gly Pro Thr Arg Ser Trp Ser Pro Tyr
1               5                   10                  15


<210>  5
<211>  25
<212>  PRT
<213>  Artificial sequence

<220>
<223>  Artificial sequence

<400>  5


<210>  6
<211>  17
<212>  PRT
<213>  Artificial sequence

<220>
<223>  Artificial sequence

<400>  6

Met Arg Trp Tyr Arg Gln Ala Pro Gly Lys Gln Arg Glu Leu Val Ser
1               5                   10                  15


His




<210>  7
<211>  39
<212>  PRT
<213>  Artificial sequence

<220>
<223>  Artificial sequence

<400>  7

Thr Tyr Tyr Ala Asp Ser Val Lys Gly Arg Phe Thr Ile Ser Arg Asp
1               5                   10                  15

Asn Ser Lys Asn Thr Leu Tyr Leu Gln Met Asn Ser Leu Arg Ala Glu
            20                  25                  30

Asp Thr Ala Val Tyr Tyr Cys
        35

<210>  8
<211>  11
<212>  PRT
<213>  Artificial sequence

<220>
<223>  Artificial sequence

<400>  8

Trp Gly Gln Gly Thr Leu Val Thr Val Ser Ser
1               5                   10

<210>  9
<211>  8
<212>  PRT
<213>  Artificial sequence

<220>
<223>  Artificial sequence

<400>  9

Gly Phe Thr Phe Ser Asn His Val
1               5

<210>  10
<211>  9
<212>  PRT
<213>  Artificial sequence

<220>
<223>  Artificial sequence

<400>  10

Ile Ser Gln Thr Gly Thr Asn Ser Asp
1               5

<210>  11
<211>  16
<212>  PRT
<213>  Artificial sequence

<220>
<223>  Artificial sequence

<400>  11

Asn Ala Val Arg Gly Arg Arg Asn Asp Gly Ser Phe Glu Asn Asp Tyr
1               5                   10                  15

<210>  12
<211>  25
<212>  PRT
<213>  Artificial sequence

<220>
<223>  Artificial sequence

<400>  12

<210>  13
<211>  17
<212>  PRT
<213>  Artificial sequence

<220>
<223>  Artificial sequence

<400>  13

Met Arg Trp Tyr Arg Gln Ala Pro Gly Lys Glu Arg Glu Met Val Ser
1               5                   10                  15

Val

<210>  14
<211>  37
<212>  PRT
<213>  Artificial sequence

<220>
<223>  Artificial sequence

<400>  14

Tyr Ala Asp Ser Val Lys Gly Arg Phe Thr Ile Ser Arg Asp Asn Ser
1               5                   10                  15

Lys Asn Thr Leu Tyr Leu Gln Met Asn Ser Leu Arg Ala Glu Asp Thr
            20                  25                  30

Ala Val Tyr Tyr Cys
        35

<210>  15
<211>  11
<212>  PRT
<213>  Artificial sequence

<220>
<223>  Artificial sequence

<400>  15

Trp Gly Gln Gly Thr Leu Val Thr Val Ser Ser
1               5                   10

## Description

### FIELD OF THE INVENTION

The present invention refers to the medical field. Particularly, it refers to the treatment of fungal infections or diseases by using specific affinity reagents, such as nanobodies, which specifically bind fungal β-1,3-glucanosyltransferase gel4 or orthologs.

### STATE OF THE ART

Over 300 million people suffer fungal infections each year and up to ∼1.6 million people -mostly immunocompromised patients- die annually of invasive fungal diseases, reaching more deaths per year than HIV, tuberculosis or malaria. Some of these diseases, such as chronic pulmonary/invasive aspergillosis and meningoencephalitis, caused by *Aspergillus fumigatus* and *Cryptococcus neoformans,* respectively, account for up to ∼1.2 million deaths annually. These examples illustrate the clinical importance of fungi and their diversity in terms of natural biological niches, modes of transmission, and clinical symptoms of their diseases. Treatments for invasive fungi are limited to polyenes, azoles, and echinocandins. However, the emerging and frequent resistance to current agents, as well as their toxicity, prompts the development of new therapies to combat these infections. This is exemplified by *Candida auris* for which long-term viability outside the human body and its resistance to commonly used antifungals are currently a major concern.

The present invention is focused on solving the above cited problem and a new therapeutic approach is herein provided for the treatment of fungal infections or diseases, such as aspergillosis and/or cryptococcosis caused, respectively, by *Aspergillus fumigatus* and/or *Cryptococcus neoformans.*

### DESCRIPTION OF THE INVENTION

### Brief description of the invention

As cited above, a new therapeutic approach is herein provided for the treatment of fungal infections or diseases, such as aspergillosis and/or cryptococcosis caused, respectively, by *Aspergillus fumigatus* and/or *Cryptococcus neoformans.*

Particularly, single-domain nanobodies against fungal β-1,3-glucanosyltransferases, which transglycosylate cell wall β-1,3-glucan are herein reported. Crystal structures of two nanobodies (hereinafter Nb3 and Nb4) with the β-1,3-glucanosyltransferase Gel4 (gGel4) from *Aspergillus fumigatus* reveal binding to a dissimilar CBM43 domain and a highly conserved catalytic-domain active site across fungal species, respectively. Anti-Gel4 active site of Nb3 shows antifungal efficacy *in vitro* and *in vivo,* protecting worms and mice against invasive *Aspergillus fumigatus* and *Cryptococcus neoformans* infection. Altogether, our findings demonstrate that nanobodies targeting these enzymes exhibit a protective role against diverse fungal pathogens and provide a strategy to combat human invasive fungal diseases.

Thus, the inventors of the present invention propose the use of specific nanobodies directed against fungal β-1,3-glucanosyltransferase gel4 for the treatment of fungal infections or diseases, such as aspergillosis and/or cryptococcosis caused, respectively, by *Aspergillus fumigatus* and/or *Cryptococcus neoformans.* So, in other words, β-1,3-glucanosyltransferase gel4 and orthologs are herein proposed as an effective therapeutic target for the treatment of fungal infections or diseases, particularly when this therapeutic target is bound by specific nanobodies.

So, the first embodiment of the present invention refers to affinity reagents, such as nanobodies, which specifically bind fungal β-1,3-glucanosyltransferase gel4, or orthologs thereof, for use in the treatment of fungal infections or diseases.

In a preferred embodiment, the nanobodies (particularly Nb3) specifically bind the catalytic-domain of fungal β-1,3-glucanosyltransferase gel4 of SEQ ID NO: 1 (which is placed between the residues 1 and 365 of SEQ ID NO: 1), preferably the residues E49, N59, D61, R125, N158, E159, D200, D202, Y230, E231, E261, D266, Y293, E298, N300 and Y302 of the catalytic-domain of fungal β-1,3-glucanosyltransferase gel4 of SEQ ID NO: 1.

In a preferred embodiment, the nanobodies (particularly Nb3) may bind other residues around the residues cited in the previous paragraph like N124, 1123, S122, S120, N162, D163, H204, A203, D201, R206, R246, Y90 and K240 of the catalytic-domain of fungal β-1,3-glucanosyltransferase gel4 of SEQ ID NO: 1.

In a preferred embodiment, the nanobodies (particularly Nb4) specifically bind CBM43 domain (which is placed between the residues 366 and 532 of SEQ ID NO: 1), preferably the residues D127, D392, F394, D395, Y396, A399, N407, K426, Y434, K437, N438 and D448 of the of fungal β-1,3-glucanosyltransferase gel4 of SEQ ID NO: 1.

In a preferred embodiment, the nanobodies (particularly Nb4) may bind other residues around the residues cited in the previous paragraph like F447, A400, I406, L393, A129 and S408 of the CBM43 domain of fungal β-1,3-glucanosyltransferase gel4 of SEQ ID NO: 1.

In this regard, kindly find below **Table 1** wherein the fungal β-1,3-glucanosyltransferase gel4 of SEQ ID NO: 1 is represented and the residues bound by the nanobodies Nb3 and Nb4 are identified. Residues bound by Nb3 are underlined. Residues bound by Nb4 are in **bold.**

**Table 1**

| fungal β-1,3-glucanosyltransferase gel4 |
|---|
| SEQ ID NO: 1 |
| MKFVYAAAGASLVGSALATLPVIEAKGNKFFYSNNGTEFFIRGVAYQQEYQANGTSTE NSDYTDPLANVDNCKRDIPYLKQLRTNVIRTYAVDPTKDHDECMKLLDDAGIYLITDLS APSESINRADPAWNTDLYKRYTSVIDAFAKYSNVIGFFAGNEVANDNNNTNSIAYVKAA VRDMKSYIKSKDYRSSLLVGYATDDDAHIRADLADYLVCGDKESSIDMFGYNIYEWCG DSSFEKSGYKDRTEEFSKYPVPAFFSEYGCIDPKPRKFTDVAALYGPQMNDVWSGGIVY MYFQEANDYGLVSVSGDNVKTKEDFSYLSVQMQKVTATGVNSASYTASNTAVPTCPS VGAKWEASNKLPPSPNSELCDCMVETLSCTVKDSVDEKEYGDLFDYLCAAGVCGGINS NSTSGDYGAYSVCSAKQKLSFVMNQYYKKNNKAATACDFDGKAQTKKGADASGSCA SLISQAGTAGTGSVTAGATGSSGSGSASETSKGAAGVAASPMAVKVGNWQFGAYIATA LFAGVGMLVL |

In a particularly preferred embodiment, the present invention refers to any of the above nanobodies for use in the treatment of aspergillosis and/or cryptococcosis.

In a preferred embodiment, the nanobodies for use, according to any of the above embodiments, particularly Nb3, are characterized in that they comprise complementary determining regions (CDR) consisting of CDR1 of SEQ ID NO: 2, CDR2 of SEQ ID NO: 3 and CDR3 of SEQ ID NO: 4, wherein CDR1, CDR2 and CDR3 are separated by frame regions FR1 of SEQ ID NO: 5, FR2 of SEQ ID NO: 6, FR3 of SEQ ID NO: 7 and FR4 of SEQ ID NO: 8.

In a preferred embodiment, the nanobodies for use, according to any of the above embodiments, particularly Nb4, are characterized in that they comprise complementary determining regions (CDR) consisting of CDR1 of SEQ ID NO: 9, CDR2 of SEQ ID NO: 10 and CDR3 of SEQ ID NO: 11, wherein CDR1, CDR2 and CDR3 are separated by frame regions FR1 of SEQ ID NO: 12, FR2 of SEQ ID NO: 13, FR3 of SEQ ID NO: 14 and FR4 of SEQ ID NO: 15.

The second embodiment of the present invention refers to a nanobody (Nb3) characterized in that it comprises complementary determining regions (CDR) consisting of CDR1 of SEQ ID NO: 2, CDR2 of SEQ ID NO: 3 and CDR3 of SEQ ID NO: 4. In a preferred embodiment, the CDR regions CDR1, CDR2 and CDR3 are separated by frame regions FR1 of SEQ ID NO: 5, FR2 of SEQ ID NO: 6, FR3 of SEQ ID NO: 7 and FR4 of SEQ ID NO: 8.

The third embodiment of the present invention refers to a nanobody (Nb4) characterized in that it comprises complementary determining regions (CDR) consisting of CDR1 of SEQ ID NO: 9, CDR2 of SEQ ID NO: 10 and CDR3 of SEQ ID NO: 11. In a preferred embodiment, the CDR regions CDR1, CDR2 and CDR3 are separated by frame regions FR1 of SEQ ID NO: 12, FR2 of SEQ ID NO: 13, FR3 of SEQ ID NO: 14 and FR4 of SEQ ID NO: 15.

In this regard, kindly find below **Table 2** and **Table 3** wherein humanized sequences of the nanobodies Nb3 and Nb4 are represented. The humanized residues are underlined.

**Table 2**

| Nanobody Nb3 | SEQ | Humanized sequence |
|---|---|---|
| CDR1 | 2 | GNIYSTNA |
| CDR2 | 3 | ITRGGRTN |
| CDR3 | 4 | NVEVRTGPTRSWSPY |
| FR1 | 5 | EVQLVESGGGLVQPGGSLRLSCAAS |
| FR2 | 6 | MRWYRQAPGKQRELVSH |
| FR3 | 7 | TYYADSVKGRFTISRDNSKNTLYLQMNSLRAEDTAVYYC |
| FR4 | 8 | WGQGTLVTVSS |

**Table 3**

| Nanobody Nb4 | SEQ | Humanized sequence |
|---|---|---|
| CDR1 | 9 | GFTFSNHV |
| CDR2 | 10 | ISQTGTNSD |
| CDR3 | 11 | NAVRGRRNDGSFENDY |
| FR1 | 12 | EVQLVESGGGLVQPGGSLRLSCAAS |
| FR2 | 13 | MRWYRQAPGKEREMVSV |
| FR3 | 14 | YADSVKGRFTISRDNSKNTLYLQMNSLRAEDTAVYYC |
| FR4 | 15 | WGQGTLVTVSS |

The fourth embodiment of the present invention refers to a pharmaceutical composition comprising any of the above defined nanobodies and, optionally, pharmaceutically acceptable excipients or carriers.

The fifth embodiment of the present invention refers to a method for treating fungal infections or diseases, such as aspergillosis and/or cryptococcosis caused, respectively, by *Aspergillus fumigatus* and/or *Cryptococcus neoformans,* which comprises the administration of a therapeutically effective amount of the above defined nanobodies or pharmaceutical composition.

For the purpose of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of'. Thus, the phrase "consisting of" indicates that the listed elements are required or mandatory, and that no other elements may be present.
- "Pharmaceutically acceptable excipient or carrier" refers to an excipient that may optionally be included with the cell suspension of the invention and that causes no significant adverse toxicological effects to the patient.
- By "therapeutically effective dose or amount" is intended an amount that, when administered as described herein, brings about a positive therapeutic response in a subject having a fungal infection or disease. The exact amount required will vary from subject to subject, depending on the age, and general condition of the subject, the severity of the condition being treated, mode of administration, and the like. An appropriate "effective" amount in any individual case may be determined by one of ordinary skill in the art using routine experimentation, based upon the information provided herein.

### Brief description of the figures

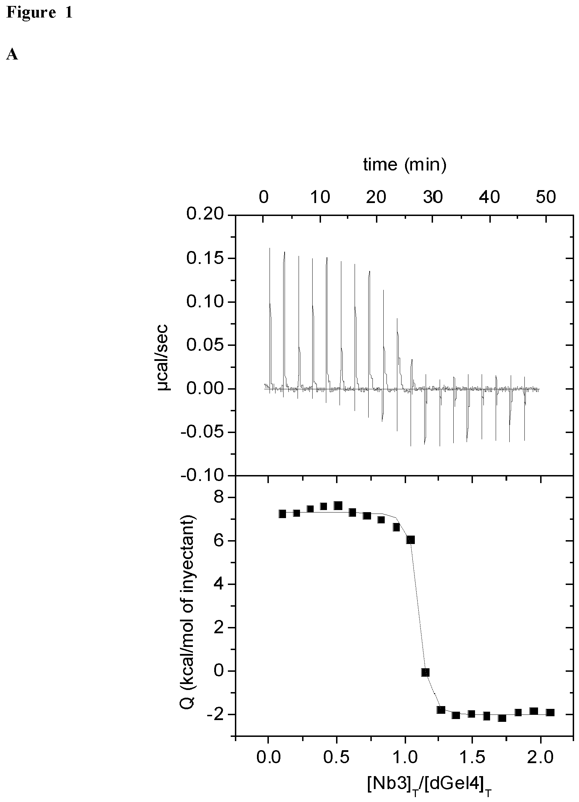

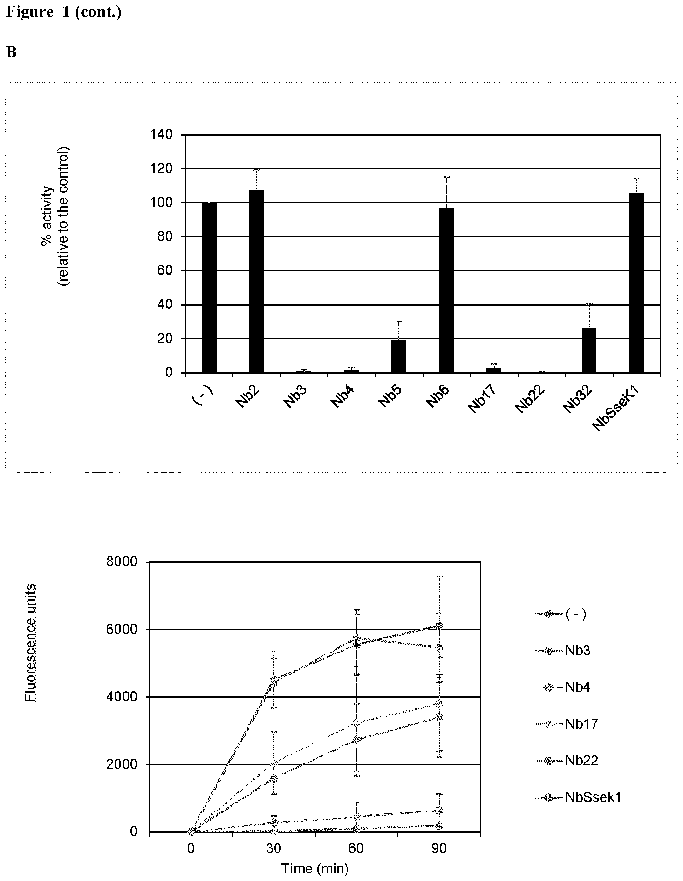

**Figure 1****. Biophysical characterization of nanobodies (Nbs). (A)** ITC data for the binding of Nb3 to dGel4. Top: raw thermogram (thermal power versus time). Bottom: binding isotherm (normalized heats versus molar ratio). **(B)** Top: inhibition of β-(1,3)-glucanosyltransglycosylase enzymatic activity of gGel4 by the Nbs at 5-fold molar excess. The inhibitory effect of Nbs is expressed as percentage of gGel4 enzymatic activity in the presence of the Nbs after 30 min of reaction relative to the control (absence of Nb). Bottom: inhibition time-course of gGel4 enzymatic activity by the indicated Nbs at 2-fold molar excess. NbSseK1 against the *Salmonella enterica* SseK1 protein unrelated to Gel4 was used as a negative control (*20*). Data are shown as mean ± standard deviation (SD) from at least three independent experiments.

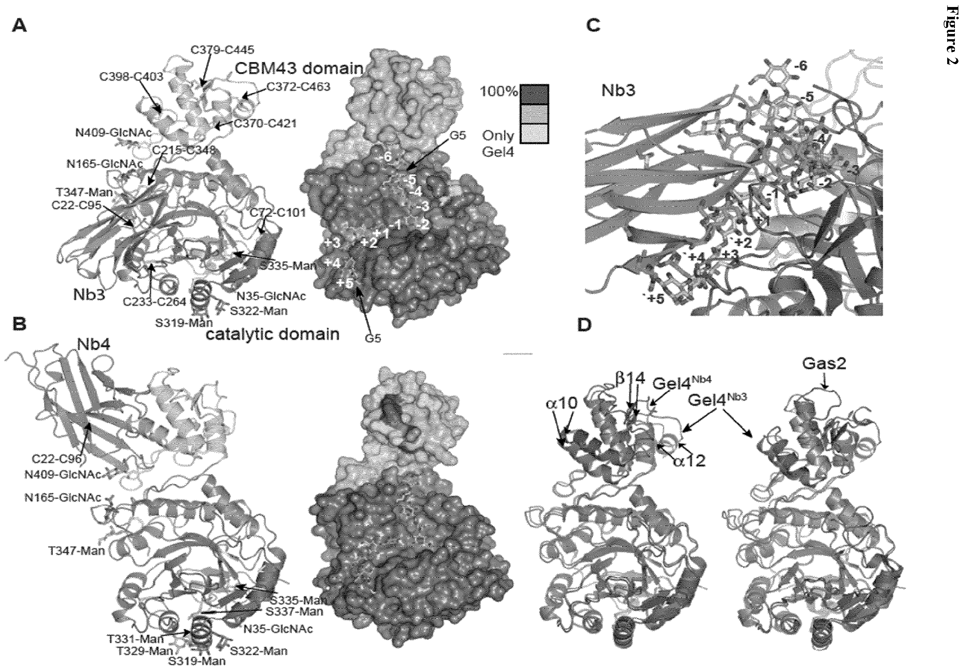

**Figure 2****. Overall structures of dGel4 complexed to Nb3 and Nb4.** (**A** and **B,** left panel) Ribbon structures of dGel4 complexed to Nb3 (top, **A**) and Nb4 (bottom, **B**). The catalytic and CBM43 domains, and the Nbs are coloured in grey, aquamarine and green, respectively. *N-*linked glycans (3 in total) and O-linked glycans (7 in total; man stands for mannose) are shown in orange carbon atoms in stick representation. Disulphide bridges are indicated as yellow sulphur atoms. (Right panels of **A** and **B**) Surface representation of dGel4-Nb3 and dGel4-Nb4 complexes, color-coded by degree of sequence conservation in the interacting regions between Gel4 and Nb3/Nb4. G5 coordinates were obtained from Gas2 complexed to G5 (PDB entry 2W62 (*15*)) and are displayed to indicate the sugar-binding subsites in Gel4. Sugar units are coloured as pink carbon atoms. **(C)** Close-up view of the dGel4-Nb3 with G5 to evidence the competitive inhibition mechanism of Nb3. **(D)** Superposition of dGel4^{Nb3} (grey) and dGel4^{Nb4} (red) structures (left panel), and dGel4^{Nb3} and Gas2 (red) structures (right panel).

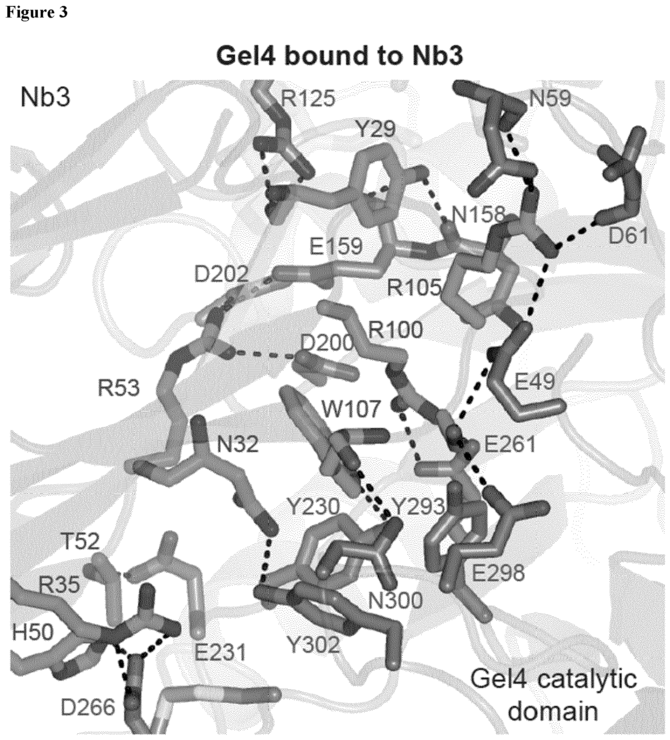

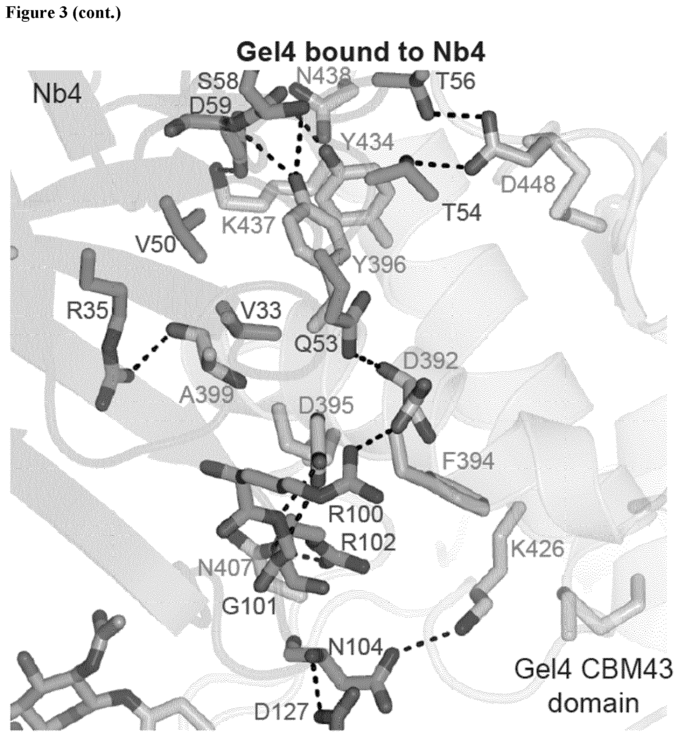

**Figure 3****. Close-up view of the interfaces of dGel4-Nb3 and dGel4-Nb4 complexes.** The residues of dGel4 catalytic and CBM43 domain, and Nb3/4, engaged in different types of interactions, are depicted as grey, aquamarine, and green carbon atoms, respectively. Hydrogen bonds are shown as dotted black lines.

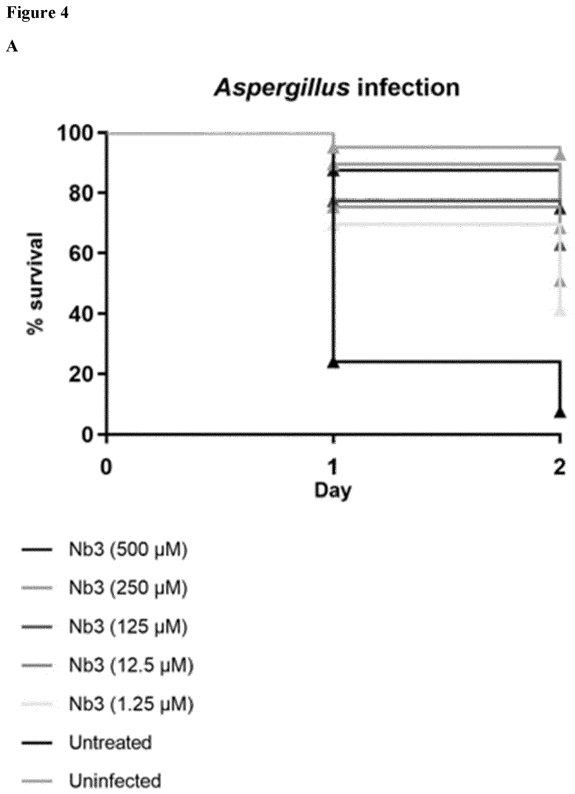

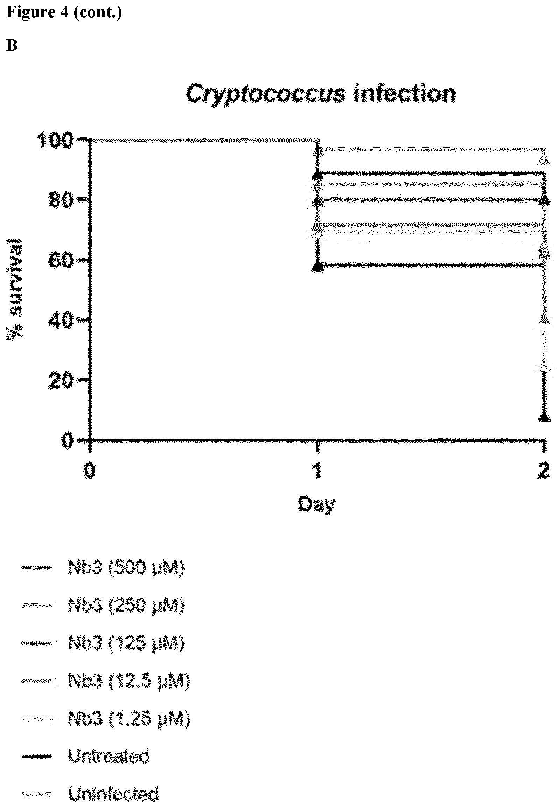

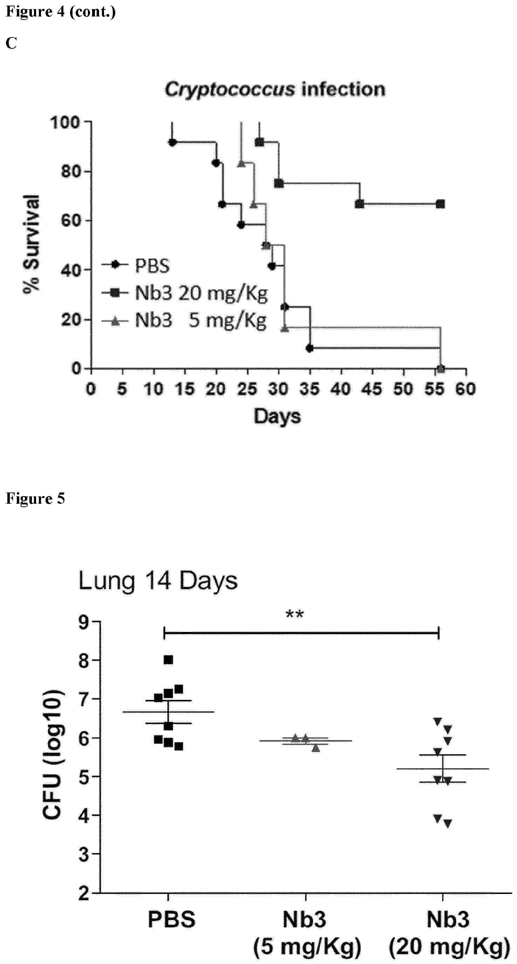

**Figure 4****. Efficacy of Nb3 in *C*. *elegans* and mice challenged with *A. fumigatus* and *C*. *neoformans.*** Survival curves of *C*. *elegans* worms treated orally with the indicated doses of recombinant Nb3 after challenge with a lethal dose (Supplementary Materials) of **(A)** *A. fumigatus* and **(B)** *C*. *neoformans.* A total of 60 worms (3 wells) were assessed for each concentration. **(C)** Effect of Nb3 at different doses on the survival of mice infected with *C*. *neoformans.*

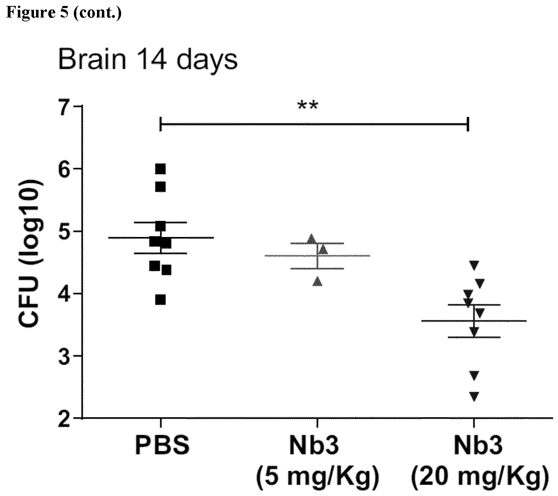

**Figure 5****. Analyses of the efficacy of Nb3 on *C*. *neoformans* colonisation of lungs and brain from infected mice.** Mice were intranasally infected with 2.5·107 CFU (colony forming units) of C. *neoformans* and subsequently treated three times a week for 2 weeks intraperitoneally with Nb3 20 mg/Kg, Nb3 5 mg/Kg or PBS as control. After 14 days of infection, mice were sacrificed, lungs **(A)** and brain **(B)** were removed, homogenised and aliquots were plated in Saboraud agar culture medium and let them grow for 1-2 days until visible colonies were observed and counted. *C. neorformans* CFUs in logarithmic decimal scale are represented in the graphs. ** p<0,01 analysed by one-way ANOVA using GraphPad software.

### Detailed description of the invention

The present invention is illustrated by means of the examples set below without the intention of limiting its scope of protection.

### Example 1. Humanization of the nanobodies.

The nanobodies were humanized following the publication [General strategy to humanize a camelid single-domain antibody and identification of a universal humanized nanobody scaffold. Vincke C, Loris R, Saerens D, Martinez-Rodriguez S, Muyldermans S, Conrath K.J Biol Chem. 2009 Jan 30;284(5):3273-84. doi: 10.1074/jbc.M806889200*. Epub 2008 Nov 14. PMID: 19010777*]. This strategy would involve the mutagenesis of 5-10 residues out of ∼140 residues.

Particularly, the sequences of Nb3 and Nb4 against Gel4 were aligned to the consensus human VH of family 3. The antigen binding loops or CDRs (underlined) were identified according to IMGT.

Comparison of each amino acid of the consensus human VH and the Nb3 or Nb4 reveals those amino acids in the framework regions that need to be substituted in the alpaca derived Nb sequence to obtain a 'humanized Nb' with lower immunogenicity risks when administered in humans.

For the humanisation, we should design the gene using the preferred codon usage for the intended expression host (*Escherichia coli* or *Pichia pastoris*). Such as it is indicated in **Table 2** and **Table 3** some residues were replaced with the human consensus sequence amino acid at specific positions. Since all amino acids that are directly involved in antigen recognition are maintained as well as all amino acids that are important for the scaffold folding of a VH and VHH, it is anticipated that this chimeric construct will bind with same affinity to its antigen as the original Nb.

### Example 2. Nanobodies obtaining and inhibitory capacity of the nanobodies.

Nbs were obtained after immunizing a llama with *A. fumigatus* deglycosylated Gel4 (dGel4). dGel4 crossreactive Nbs were isolated by phage display, rendering 8 Nbs grouped in 3 families. The binding affinities of these 8 Nbs against the glycosylated (gGel4) and deglycosylated (dGel4) forms of Gel4 were evaluated by isothermal titration calorimetry (ITC) **(****Figure 1A****).** All Nbs exhibited ∼1:1 binding to either gGel4 or dGel4, with affinity constants in the low and medium nM range. The affinities of the Nbs were better for dGel4 than gGel4 (*K_{D}*s range between 0.24-19.74 and 1.56-575 nM, respectively), implying that glycans present in gGel4 might impede optimal Nb recognition. In particular, these differences in *K_{D}*s were mostly notable for Nb4/6/22/32, while Nb2/3/5/17 showed small or no differences in the *K_{D}*s between either forms. Detailed analysis of the thermodynamic parameters of the interaction showed that the binding of Nb3 to either enzymatic form and Nb6 to gGel4 were largely entropy-driven (-TΔS), while the other Nbs were favoured by a gain in enthalpy (ΔH), implying distinct interaction behaviours.

To evaluate the inhibitory capacity of the Nbs, the transglycosylase activity of gGel4 was measured in a time-course assay in the presence of the Nbs using laminarin as the glucanosyl donor and sulphorhodamine-labeled laminarihexaose as the acceptor substrate. A 5-fold molar ratio Nbs over gGel4 (5:1 ratio) showed that Nbs 3/4/5/17/22/32 inhibited gGel4 activity **(****Figure 1B****;** top panel). At lower molar ratio (2:1), Nb3 and Nb4 were the most potent ones, followed to a lesser extent by Nb22 and Nb17 **(****Figure 1B****;** bottom panel). As expected by the highly similar Nb3's *K_{D}*s, there were no differences on inhibition of either gGel4 or dGel4 by Nb3. Similarly, Nb4's IC₅₀s were very similar, despite significant differences in Nb4's *K_{D}*s, implying that there was no effect of the gGel4 glycans on Nb4 inhibition activity. The activity of other Gel4 orthologs, namely Phr1 and Phr2 from *C*. *albicans,* or Gas1 and Gas2 from *S. cerevisiae,* was also evaluated in the presence of Nb3 and Nb4. While Nb3 inhibited moderately (5:1 ratio) and significantly (200:1 ratio) Gas2 and Phr2, Nb4 only inhibited Phr2. These results suggest that Nb3 and Nb4 are fairly selective inhibitors of Gel4, although Nb3 can inhibit more broadly other Gel4 orthologs at higher concentrations.

### Example 3. Structures of dGel4 complexed to Nb3 and Nb4.

To elucidate the molecular basis of Gel4 inhibition by the most potent Nbs, we determined the crystal structures of dGel4 complexed to Nb3 and Nb4 at 2.05-Å and 1.90-Å resolution, respectively **(****Figure 2** A and B). The structure of dGel4 shows a compact structure with the typical (β/α)₈ catalytic core and the cysteine-rich CBM43 domain located at the N- and C-terminal regions, respectively. While Nb4 binds to the CBM43 domain burying a Gel4 surface area (SA) of 3,870 A², Nb3 binds to a shallow active site located in the catalytic domain, burying a larger SA of 4,860 A². Particularly, Nb3 occupies the -6 to -1 and +1 to +5 sugar-binding subsites, impeding binding to β-1,3-glucan and in turn transglycosylation **(****Figure 2****,** A and C). The Nb4 binding to CBM43 and its inhibitory properties demonstrate that this domain, distantly located from the catalytic machinery, likely interacts with β-1,3-glucan and in turn is critical for transglycosylation. Hence, Nb4 as well as Nb5/17/22/32, all displaying enthalpy-driven binding, might also compete with β-1,3-glucan sugar units for the association to the CBM43 domain. These data suggest that our Nbs might adopt at least two different binding modes inhibiting Gel4. Note also that the Gel4 catalytic domain residues interacting with Nb3 are more conserved across orthologs than the CBM43 domain residues interacting with Nb4 **(****Figure 2** A and B).

A comparison of the overall crystal structures of Gas2-laminaripentaose (G5) (*15*) and dGel4-Nb3 complexes reveals that they share most of the secondary structure elements and superimpose fairly well (root mean square deviation (RMSD) of 1.39 Å on 403 equivalent Cα atoms **(****Figure 2D****).** However, the RMSDs slightly increase when crystal structures of Gas2-G5 and dGel4-Nb3 complexes are compared to Gel4-Nb4 complex (RMSDs of 1.76 Å and 1.56 Å on 398 and 408 Cα atoms, respectively), due to conformational changes induced by Nb4 binding to Gel4, which are located in the CBM43 α10/α12/β14 and loops **(****Figure 2D****).**

A close-up view of the dGel4-Nb3/4 interfaces confirms that the three hypervariable loops, known as H1, H2, and H3, are engaged in recognition of dGel4. Nb3 contacted mostly conserved residues of the catalytic domain (9/15 residues are completely conserved with Gel4 orthologs; **Figure 3****),** such as Gel4 residues E49, N59, D61, R125, N158, E159, D200, D202, Y230, E231, E261, D266, Y293, E298, N300 and Y302. Out of these residues, N158/E159/E261 and Y230/Y293/Y302, based on site-directed mutagenesis and computational studies on equivalent residues in Gas2, are critical residues for catalysis and recognition of sugar-units, respectively. In contrast, Nb4 contacted mostly specific residues of the Gel4 CBM43 domain (only few residues were partly conserved with other Gel4 orthologs; **Figure 3**), such as Gel4 residues D127, D392, F394, D395, Y396, A399, N407, K426, Y434, K437, N438, and D448. In both complexes, most of the interactions were through hydrogen bonds and salt bridges and were established between side chains. However, hydrophobic interactions were also present, though these were in the minority in the dGel4-Nb3 complex. The residues of Nb3 engaged in dGel4 recognition are mostly unique for this Nb, while the residues of Nb4 engaged with dGel4 are mostly conserved among the Nbs 2/4/5/6/17 and 32, implying that they might adopt similar binding modes as suggested above. In contrast, Nb22, which mostly does not share the residues of Nb3 and Nb4 interacting with dGel4, might adopt a different binding mode to those of Nb3 and Nb4. In addition, the unique thermodynamic profile exhibited by Nb3 might be due to the release of a vast number of water molecules from the large active site of Gel4 upon Nb3 binding, promoting a favourable desolvation entropy.

### Example 4. Efficacy of Nb3 in C. elegans and mice challenged with A. fumigatus and C. neoformans.

Having established that Nb3 might be a pan-β-1,3-glucanosyltransferase inhibitor by targeting a highly conserved catalytic-domain active site, we next questioned whether Nb3 could be effective in inhibiting *A. fumigatus* and *C*. *neoformans.* Although there are no studies of these enzymes in *C*. *neoformans,* we performed a BLAST search using the Gel4 catalytic domain against the *C*. *neoformans* genome, and found only one member in this fungus, which we named hereafter as *Cn*Gel (41% identity with Gel4). Nb3 inhibited *A. fumigatus* growth well, and surprisingly, strongly inhibited *C*. *neoformans* cell growth

To explore if Nb3 could potentially be useful as part of a combinatorial therapy, growth inhibition due to Nb3 in combination with caspofungin (CSF) at sub-inhibitory concentrations, was analysed. Strikingly, combination of Nb3 and CSF led to a potent synergistic inhibitory effect against *C*. *albicans* (IC₅₀s = 7.11 ± 5). The combination of variable Nb3 concentrations with CSF rendered a synergistic inhibitory effect on both fungi. In fact, the IC₅₀s values decreased 2.8 fold*^{A. fumigatus}* and 3.8-fold*^{C.neoformans}* compared to the corresponding ones for Nb3 alone.

On the other hand, since Voriconazole (VOR) or analogs, either alone or in combination with other drugs are currently the best choice of treatment against invasive aspergillosis and cryptococcosis, we determined the effect of the combination of a sub-inhibitory concentration of VOR with variable Nb3 concentrations in *A. fumigatus* and VOR-resistant *C*. *neoformans* growth. We found a synergistic inhibition of Nb3 and VOR against *C*. *neoformans.* Besides, the inhibitory effect of our Nb3 with VOR was superior to that of the voriconazole alone. These results suggest that Nb3 or its combination with CSF and VOR might bring therapeutic benefits to combat these opportunistic pathogens or their drug-resistant forms.

### Example 5. In vivo protection conferred by the nanobodies of the invention.

To assess *in vivo* protection, we evaluated the efficacy of oral administration of Nb3 on *C*. *elegans* challenged with *A. fumigatus* and *C*. *neoformans.* All animals died after 48 h of infection. Treatment with all concentrations of Nb3 improved animal survival being statistically significant (Mantel-Cox test p-value <0.0001). Specifically, Nb3 at 12.5 and 500 µM resulted in ∼50% and ∼80% survival of infected *C*. *elegans* by both fungi, respectively **(****Figure 4****,** A and B). This demonstrates that low concentrations are enough to protect half of the worms from infection while higher concentrations are required to reach a more optimal protection. This high concentration was required to account for the possibility that Nb3 might get partly degraded by the *C*. *elegans* digestive system. Despite that overdose, Nb3 was safe in *C*. *elegans,* in contrast to the inherent toxicity of other drugs as VOR or CSF. Thus, the effect of both drugs was not tested in this model.

Prompted by the encouraging results in worms, we evaluated the protective role of Nb3 on mice challenged with *C*. *neoformans* by using an intraperitoneal administration. Nb3 was able to inhibit, at different doses, mice infection caused by *Cryptococcus neoformans* **(****Figure 4C****).**

### Example 6. Efficacy of Nb3 on C. neoformans colonisation of lungs and brain from infected mice.

The efficacy of the nanobody Nb3 against *C*. *neoformans* colonisation of lungs and brain of infected mice was assessed. Mice were intranasally infected with 2.5·107 CFU (colony forming units) of *C*. *neoformans* and subsequently treated three times a week for 2 weeks intraperitoneally with Nb3 20 mg/Kg, Nb3 5 mg/Kg or PBS as control. Such as it is shown in **Figure 5****,** after 14 days of infection, mice were sacrificed, lungs **(****Figure 5A****)** and brain **(****Figure 5B****)** were removed, homogenised and aliquots were plated in Saboraud agar culture medium and let them grow for 1-2 days until visible colonies were observed and counted. As shown in **Figure 5****,** a statistically significant reduction of the CFU is observed both in brain and lungs when Nb3 nanobody was administered at different doses.

## Claims

1. Affinity reagents such as nanobodies which specifically bind the fungal β-1,3-glucanosyltransferase gel4, or orthologs thereof, for use in the treatment of fungal infections or diseases.

2. Nanobody for use, according to claim 1, **characterized in that** it specifically binds the catalytic-domain of fungal β-1,3-glucanosyltransferase gel4 which is placed between the residues 1 and 365 of SEQ ID NO: 1.

3. Nanobody for use, according to claim 2, **characterized in that** it specifically binds the residues E49, N59, D61, R125, N158, E159, D200, D202, Y230, E231, E261, D266, Y293, E298, N300 and Y302 of the catalytic-domain of fungal β-1,3-glucanosyltransferase gel4 of SEQ ID NO: 1.

4. Nanobody for use, according to claim 3, **characterized in that** it further binds the residues N124, 1123, S122, S120, N162, D163, H204, A203, D201, R206, R246, Y90 and K240 of the catalytic-domain of fungal β-1,3-glucanosyltransferase gel4 of SEQ ID NO: 1.

5. Nanobody for use, according to claim 1, **characterized in that** it specifically binds the CBM43 domain of fungal β-1,3-glucanosyltransferase gel4 which is placed between the residues 366 and 532 of SEQ ID NO: 1.

6. Nanobody for use, according to claim 5, **characterized in that** it specifically binds the residues D127, D392, F394, D395, Y396, A399, N407, K426, Y434, K437, N438 and D448 of the CBM43 domain of fungal β-1,3-glucanosyltransferase gel4 of SEQ ID NO: 1.

7. Nanobody for use, according to claim 6, **characterized in that** it further binds the residues F447, A400, 1406, L393, A129 and S408 of the CBM43 domain of fungal β-1,3-glucanosyltransferase gel4 of SEQ ID NO: 1.

8. Nanobody for use, according to any of the previous claims, in the treatment of aspergillosis and/or cryptococcosis.

9. Nanobody for use, according to claims 2 to 4, **characterized in that** it comprises complementary determining regions (CDR) consisting of CDR1 of SEQ ID NO: 2, CDR2 of SEQ ID NO: 3 and CDR3 of SEQ ID NO: 4.

10. Nanobody for use, according to claim 9, **characterized in that** CDR1, CDR2 and CDR3 are separated by frame regions FR1 of SEQ ID NO: 5, FR2 of SEQ ID NO: 6, FR3 of SEQ ID NO: 7 and FR4 of SEQ ID NO: 8.

11. Nanobody for use, according to claims 5 to 7, **characterized in that** it comprises complementary determining regions (CDR) consisting of CDR1 of SEQ ID NO: 9, CDR2 of SEQ ID NO: 10 and CDR3 of SEQ ID NO: 11.

12. Nanobody for use, according to claim 11, **characterized in that** CDR1, CDR2 and CDR3 are separated by frame regions FR1 of SEQ ID NO: 12, FR2 of SEQ ID NO: 13, FR3 of SEQ ID NO: 14 and FR4 of SEQ ID NO: 15.

13. Nanobody **characterized in that** it comprises complementary determining regions (CDR) consisting of CDR1 of SEQ ID NO: 2, CDR2 of SEQ ID NO: 3 and CDR3 of SEQ ID NO: 4; or consisting of CDR1 of SEQ ID NO: 9, CDR2 of SEQ ID NO: 10 and CDR3 of SEQ ID NO: 11.

14. Nanobody, according to claim 13, wherein the CDR1 of SEQ ID NO: 2, CDR2 of SEQ ID NO: 3 and CDR3 of SEQ ID NO: 4 are separated by frame regions FR1 of SEQ ID NO: 5, FR2 of SEQ ID NO: 6, FR3 of SEQ ID NO: 7 and FR4 of SEQ ID NO: 8; or wherein the CDR1 of SEQ ID NO: 9, CDR2 of SEQ ID NO: 10 and CDR3 of SEQ ID NO: 11 are separated by frame regions FR1 of SEQ ID NO: 12, FR2 of SEQ ID NO: 13, FR3 of SEQ ID NO: 14 and FR4 of SEQ ID NO: 15.

15. Pharmaceutical composition comprising the nanobodies of claims 13 or 14 and, optionally, pharmaceutically acceptable carriers or excipients.
